# EUROPEAN PATENT SPECIFICATION

(11) **EP 2 653 872 B1**
(45) Date of publication and mention of the grant of the patent: **22.02.2017**
(21) Application number: 11849353.5
(22) Date of filing: 05.12.2011
(51) Int. Cl.: G01N 33/68, G01N 33/15, G01N 33/50, G01N 33/53

(54) **BIOMARKER FOR AMYLOID-BETA -RELATED NEUROLOGICAL DISORDERS**
BIOMARKER FÜR AMYLOID-BETA-VERMITTELTE NEUROLOGISCHE ERKRANKUNGEN
BIOMARQUEURS POUR TROUBLES NEUROLOGIQUES LIÉS AU PEPTIDE BETA -AMYLOÏDE

(30) Priority: 16.12.2010 JP 2010280331
(43) Date of publication of application: 23.10.2013
(73) Proprietor: Sapporo Medical University, Sapporo-shi, Hokkaido 060-8556 (JP)
(72) Inventor: KOKAI, Yasuo, Sapporo-shi, Hokkaido 060-8556 (JP); SOHMA, Hitoshi, Sapporo-shi, Hokkaido 060-8556 (JP); IMAI, Shinichi, Sapporo-shi, Hokkaido 060-8556 (JP); MATSUMOTO, Kayo, Sapporo-shi, Hokkaido 060-8556 (JP); KIMURA, Michitoshi, Sapporo-shi, Hokkaido 060-8556 (JP)
(74) Representative: Schwarz & Partner
(86) International application number: PCT/JP2011/078036
(87) International publication number: WO 2012/081433

(56) References cited:
- WO-A2-2006/014638
- SILVESTRE J-S ET AL: "Lactadherin promotes VEGF-dependent neovascularization", 1 May 2005 (2005-05-01), NATURE MEDICINE, NATURE PUBLISHING GROUP, NEW YORK, NY, US, PAGE(S) 499 - 506, XP002356215, ISSN: 1078-8956 * page 505, left-hand column, paragraph 3 * * page 505, right-hand column, paragraph 1 *
- BODDAERT J. ET AL.: 'Evidence of a role for lactadherin in Alzheimer's disease.' AM.J. PATHOL. vol. 170, no. 3, 2007, pages 921 - 9, XP055112517
- SPITSBERG VL.: 'Invited review: Bovine milk fat globule membrane as a potential nutraceutical.' J DAIRY SCI. vol. 88, no. 7, 2005, pages 2289 - 94, XP002436135
- FULLER A.D. ET AL.: 'MFG-E8 Regulates Microglial Phagocytosis of Apoptotic Neurons' J NEUROIMMUNE PHARMACOL. vol. 3, no. 4, 2008, pages 246 - 256, XP055112523
- RAYMOND A. ET AL.: 'SED1/MFG-E8: a bi-motif protein that orchestrates diverse cellular interactions.' J CELL BIOCHEM. vol. 106, no. 6, 2009, pages 957 - 66, XP055112525

## Description

### TECHNICAL FIELD

The present invention relates to a method for diagnosing Alzheimer's disease and the use of a diagnostic reagent and of a test kit for this method.

### BACKGROUND ART

Alzheimer's disease is a progressive neurodegenerative disease and characterized by accumulation of an insoluble fibrous protein called β-amyloid in the brain. Various methods are proposed for diagnosing Alzheimer's disease, including detection of a particular protein in cerebrospinal fluid, detection of a particular protein or lipoprotein in the blood or detection of genetic mutation such as genetic polymorphism. However, a simple and effective diagnosis method using peripheral blood has not yet been put into practical use.

Non Patent Literature 1: J Neuroimmune Parmacol (2008) 3: 246-256. Silvestre et al., Nature Medicine, 11 (2005), 499 - 506 discloses compositions comprising an anti-MFG-E8 antibody and a reagent for detecting a MFG-E8 protein bound to the anti-MFG-E8 antibody.

### DISCLOSURE OF THE INVENTION

An object of the present invention is to provide a novel biomarker for diagnosing amyloid β-related neuropathy such as Alzheimer's disease.

The present inventors have carried out screening of a group of proteins present in a culture supernatant of a primary culture of mouse nerve cells, which are a model of dementia, using magnetic nanoliposomes that mimic a brain biomembrane, and have identified a protein MFG-E8 which binds to phosphatidylserine in a Ca²⁺ dependent manner. The present inventors further found that the level of the MFG-E8 protein is increased in patients with Alzheimer's disease and thus MFG-E8 may represent a biomarker for Alzheimer's disease.

Thus the present invention provides a method for diagnosing Alzheimer's disease comprising measuring the level of a MFG-E8 protein in a blood, serum or plasma sample obtained from a human or animal subject, wherein the measured value is used as a marker for diagnosis of Alzheimer's disease. Preferably, the diagnosis of Alzheimer's disease is made at a stage where an accumulation of an amyloid β protein has not been advanced. Preferably, the level of the MFG-E8 protein is measured with an anti-MFG-E8 antibody.

The present invention also provides the use of a diagnostic reagent for Alzheimer's disease comprising an anti-MFG-E8 antibody. The present invention further provides the use of a test kit for Alzheimer's disease comprising an anti-MFG-E8 antibody and a reagent for detecting a MFG-E8 protein bound to the anti-MFG-E8 antibody.

In another aspect, the present invention provides a method for selecting a candidate substance for a therapeutic agent for Alzheimer's disease. The method comprises the steps of administering a test substance to a model cell or a model animal of Alzheimer's disease; measuring the amount of a MFG-E8 protein in a culture medium of the model cell or a sample obtained from the model animal; and selecting the test substance as a candidate substance for a therapeutic agent for Alzheimer's disease when the amount of the MFG-E8 protein is decreased with administration of the test substance compared to the amount thereof without administration.

According to the present invention, the onset of Alzheimer's disease can be diagnosed by a simple method.

### BRIEF DESCRIPTION OF THE DRAWINGS

Fig. 1 shows the structures of murine and human MFG-E8s;
Fig. 2 shows the results of SDS-PAGE of a fraction from a culture supernatant which bound to nanoliposomes in a Ca²⁺ dependent manner;
Fig. 3 shows the results of the measurement of the expression level of MFG-E8 by Western blotting; and
Fig. 4 shows a ROC curve of plasma MFG-E8 concentration for healthy elderly subjects vs. AD patients.

### PREFERRED EMBODIMENTS OF THE INVENTION

The present invention features a method for diagnosing Alzheimer's disease by measuring the expression level of MFG-E8 in a subject sample such as blood of a human subject.

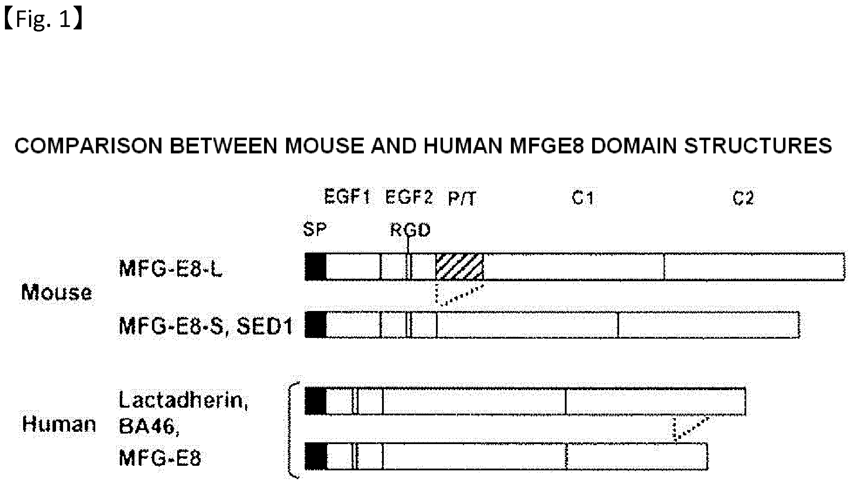

MFG-E8 (Milk fat globule EGF factor 8) is a secretory protein also referred to as Lactadherin, P47, Mfgm or SED1 (GenBank No. NP_032620.2. IPI No. IPI00788387.1) and has two functional domains (N-terminal EGF domain and C-terminal C2 domain). There are isoforms of L-type (72 kDa, 61 kDa) and S-type (56 kDa, 48 kDa) and it is reported that the L-type is a secretory form (Fig. 1).

MFG-E8 contributes to phagocytosis of apoptotic cells in many tissues. It is known that MFG-E8 is an important regulatory factor for phagocytosis of apoptotic cells, which transmits the eat-me signal of apoptotic cells to macrophages, promotes phagocytotic action of macrophages and competitively inhibits the phagocytotic action at an excess amount in the peripheral blood. MFG-E8 binds to PS on apoptotic cells as well as to integrin on macrophages, and is involved in removal of apoptotic cells by macrophages, mammary gland involution after the lactation period, phagocytosis of photoreceptor outer segment membranes after detachment of retinal pigment epithelia, and clearance of debris of atherosclerotic intravascular apoptotic cells. MFG-E8 is also known to promote intercellular interactions and is involved in joining of sperms and eggs, maintenance of epididymal epithelia, repair of intestinal epithelia, promotion of ductal branching, angiogenesis, and promotion of exosome function of dendric cells.

Fuller et al. (J Neuroimmune Pharmacol (2008) 3: 246-256) studied the kinetics of MFG-E8 using a microglia-derived cell line and reported that microglia cells produce MFG-E8 in the brain and MFG-E8 may possibly recognize apoptotic neuroblastoma cells and play a role in phagocytosis. This may correspond to the function in which macrophages produce MFG-E8 in the peripheral blood and MFG-E8 recognizes apoptotic cells and transmits the information to macrophages. Fullers et al. further carried out experiments and found that TG mice (Alzheimer model mice) having the APP gene of human familial Alzheimer's disease have a decreased level of MFG-E8 in the brain compared to normal mice. In this context, it is interpreted that disruption in control of MFG-E8 may play some role in progression of Alzheimer's disease. Similarly, Boddaert J et al. (Am J Pathol (2007) 170: 921-929) also showed the decreased MFG-E8 mRNA expression in the brain of subjects with Alzheimer's disease compared to the brain of age-matched subjects.

To the contrary, the present inventors have found in an immunostaining experiment that no accumulation of Aβ and MFG-E8 was observed in the brain of normal mice (12-month old) and MFG-E8 is accumulated at the same site as the Aβ accumulation site in the brain of Alzheimer's disease model mice (12-month old). This means that the present inventors' results are completely opposite to the results of Fuller et al. and Boddaert et al., the reason for which may be the difference in antibodies used; however the details are not clear. Prior to this finding, the present inventors also found that the level of MFG-E8 in the peripheral blood was also significantly high in patients with Alzheimer's disease, as specifically shown in Examples hereinbelow. The observed MFG-E8 in the peripheral blood may also possibly be the MFG-E8 which has been accumulated at the same site as the Aβ accumulation site and leaked. Some studies suggested that the permeability from blood to brain is elevated at the blood-brain barrier in patients with Alzheimer's disease; however the extent is limited. Permeability from brain to blood remains unclear. Therefore it is unlikely that the MFG-E8 observed at the Aβ accumulation site directly contributes to the concentration of MFG-E8 in the blood beyond the blood-brain barrier. Detailed mechanism of the correlation between the increased level of MFG-E8 and Alzheimer's disease found in the present invention is still unclear.

The subject sample to be analyzed for the level of the MFG-E8 protein may includes body fluid, blood, serum, and plasma obtained from a human or animal subject. From the reason described above, cerebrospinal fluid is not suitable as the sample.

The amount of the MFG-E8 protein in the sample obtained from the subject can be measured by immunoassays well known in the art using an anti-MFG-E8 antibody. The anti-MFG-E8 antibody may be monoclonal or polyclonal. Various anti-MFG-E8 polyclonal and monoclonal antibodies are commercially available and can be used for the present invention. Alternatively the antibody may be prepared according to the method well known in the art.

The MFG-E8 protein in the sample obtained from a human or animal subject is measured by an immunoassay using the anti-MFG-E8 antibody. The measurement may be qualitative or quantitative. The expression level of MFG-E8 in the sample obtained from a subject may be immunologically assayed by, for example, radioimmunoassay, ELISA, immunochromatography, immunoprecipitation, immune agglutination, and Western blotting. Alternatively, the measurement may be carried out with a biosensor utilizing the surface plasmon resonance phenomenon.

As a typical example, sandwich ELISA can be carried out as follows. Plasma prepared from peripheral blood taken from a subject is added to a plate or chip onto which an anti-MFG-E8 antibody has been immobilized and incubated for an appropriate period of time. The plate or chip is washed to remove unbound components followed by addition of a second anti-MFG-E8 antibody. The second antibody may be labeled with a detectable label, such as an enzyme, a fluorescence dye, a chemiluminescence substance, biotin, a radioactive substance. After incubation for an appropriate period of time, the plate or chip is washed and measured for fluorescence, luminescence, or radioactivity to detect the label. Alternatively after binding of the anti-MFG-E8 antibody, a secondary antibody (e.g. goat anti-mouse antibody) may be added in order to enhance the signal. The secondary antibody may be labeled with a detectable label, such as an enzyme, a fluorescence dye, a chemiluminescence substance, biotin, a radioactive substance. In this way, the amount of the MFG-E8 protein in the plasma obtained from a subject can be measured.

In another aspect, the MFG-E8 protein can be detected by a detection method utilizing agglutination reaction. In the method, an anti-MFG-E8 antibody may be attached to a carrier, e.g. latex particles for detecting MFG-E8. Latex particles carrying the anti-MFG-E8 antibody are mixed with a sample and incubated for a certain period of time. The particles will agglutinate when the sample contains MFG-E8. The extent of agglutination may be observed visually or quantified with a spectrophotometer to detect MFG-E8 in the sample.

The present invention also provides a diagnostic reagent for Alzheimer's disease comprising an anti-MFG-E8 antibody. The diagnostic reagent for Alzheimer's disease of the present invention may be provided in the form of a test kit. The test kit comprises a reagent for detecting MFG-E8, e.g. the anti-MFG-E8 antibody as the active substance. The kit may also comprise appropriate reagents required for measurements, for example, buffers, diluents, reaction terminating solutions, washing solutions and control standards.

In the present invention, the amount of the MFG-E8 protein thus measured may be used as a marker for Alzheimer's disease. Specifically, the amount of the MFG-E8 protein is measured on a sample obtained from a human or animal subject, the resulting measured value is compared to a measured value obtained from a healthy control. When the measured value of the subject is higher than that of the control, the human or animal subject is diagnosed to have a high possibility of having Alzheimer's disease. The course of Alzheimer's disease may also be monitored with the method of the present invention. The amount of the MFG-E8 protein is measured in samples obtained from a human or animal subject at multiple time points, and an increase in the resulting measured values with time indicates progression of Alzheimer's disease and a decrease indicates amelioration of Alzheimer's disease. The course of treatment may be monitored by the method in order to determine the therapeutic efficacy.

According to the method of the present invention, Alzheimer's disease can be diagnosed based on the MFG-E8 protein level in the blood as a marker without collecting cerebrospinal fluid. As shown in Examples hereinbelow, the expression level of MFG-E8 in the plasma of patients with Alzheimer's disease is significantly increased compared to healthy control subjects. The diagnostic method of the present invention is useful for an early diagnosis of Alzheimer's disease (diagnosis at an initial stage where an accumulation of the amyloid β protein has not been advanced), a definitive diagnosis of the onset, a monitoring of the course of the disease, determination of the therapeutic efficacy and prognosis.

In another aspect of the present invention, a candidate substance for a therapeutic agent for Alzheimer's disease can be screened based on the expression level of the MFG-E8 protein as a marker. Screening is carried out by administering a test substance to a model cell or a model animal for Alzheimer's disease, e.g. a mouse nerve cell primary culture or a TG mouse administered with the cytotoxic Aβ₄₂ peptide, and measuring the level of the MFG-E8 protein in a culture medium of the model cell or a sample obtained from the model animal. The test substance used for the screening may include various synthetic chemicals and antibodies. The test substance can be obtained from, for example, libraries such as various synthetic or natural chemical libraries, combinatorial libraries, oligonucleotide libraries and peptide libraries. The test substance may be extracts from natural substances such as bacteria, fungi, algae, plants and animals or partially purified products thereof. The test substance can be selected as the candidate substance for a therapeutic agent for Alzheimer's disease when the level of the MFG-E8 protein is decreased with administration of the test substance compared to the level thereof without administration. Thus the test method of the present invention provides a platform for development of novel therapeutic methods of Alzheimer's disease.

The present invention will be described in detail below by way of examples, but the invention is not limited by these examples.

### Example 1

### Investigation of biomarkers for amyloid β (Aβ) -related neuropathy

The cytotoxic Aβ₄₂ peptide was administered to a mouse and a primary culture of mouse nerve cells were used as a model of dementia. Candidates for biomarkers for amyloid β-related neuropathy were screened using magnetic nanoliposomes which mimick a brain biomembrane. Aβ₄₂ is one of the final products generated after cleavage of the amyloid precursor protein (APP; 150 kD) with three enzymes, which has the strongest cytotoxicity and has been used for various studies.

Mouse telencephalon primary culture cells (5 x 10⁶ / 10-ml dish) were stimulated with Aβ₄₂ (0.1 µM) for 24 hours. The conditions selected were at a low concentration for a short time in order to provide mild cytotoxicity as well as with assuming the state of the initial stage of Alzheimer's disease. Namely the concentration of 0.1 µM and a period of 24 hours were selected so as to avoid cellular necrosis or apoptosis. The cells without stimulation with Aβ₄₂ were used as a control. The cell culture supernatant was treated with EGTA (final concentration: 5 mM) and a certain amount thereof was pooled, which was then concentrated from 10 ml to 1 ml by ultrafiltration with an Amicon Ultra-15 3,000 NMWL. The concentrated supernatant was added with 1 mg of nanoliposomes and CaCl₂ (final concentration: 10 mM), adjusted pH with 0.5 M HEPES (pH 7.5) and allowed to react in a rotor at 4°C for 1.5 hours. After the reaction Ca²⁺ was chelated with EGTA, proteins were separated from nanoliposomes to obtain a fraction which bound to an acidic phospholipid (phosphatidylserine) in a Ca²⁺ dependent manner.

The principle and detailed procedures for the method of admixing a sample and liposomes in the presence of calcium ions and separating a liposome-binding protein with a chelating agent are disclosed in Japanese Patent Application Laid-open No. 2008-020383. The nanoliposomes are magnetic fine particles modified on the surface with a thermoresponsive polymer and a phospholipid and that can form a liposome-like structure in an aqueous solution. The principle and structure of nanoliposomes are specifically illustrated in Japanese Patent Application Laid-open No. 2010-066200. When the nanoliposomes are mixed with a sample in an aqueous solution, a phospholipid-binding component in the sample will bind to nanoliposomes, which are then collected with the aid of temperature variation and magnetism to allow simplified collection of the phospholipid-binding component.

The phospholipid used herein was phosphatidylserine. Phosphatidylserine is one of lipids constituting a cell membrane. It is usually located internally to the cell membrane while it is exposed external of the cells when the cells undergo apoptosis. Thus it is believed that apoptosis-related proteins may be identified by screening of proteins which bind to phosphatidylserine.

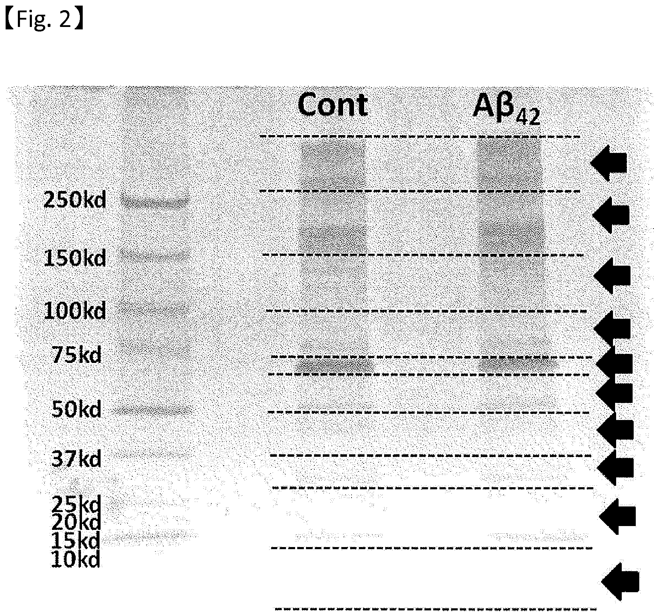

The fraction thus obtained from the culture supernatant which bound to nanoliposomes in a Ca²⁺ dependent manner was separated by one-dimensional SDS-PAGE and silver staining. The results are shown in Fig. 2.

Next, the gel was divided into 10 slices at the positions shown with the arrows in Fig. 2 and subjected to in-gel digestion with trypsin to collect peptides, which were then analyzed by mass spectrometry. Among the top 100 identified nanoliposome-binding proteins, the ones which had an increased expression level after stimulation with Aβ were analyzed, and MFG-E8 (milk fat globule-EGF factor 8 protein isoform 1) was identified as a candidate marker protein.

### Example 2

### Western blotting analysis

The difference in the expression level of MFG-E8 at the protein level was analyzed by Western blotting.

The material used was a PS 100% nanoliposome-binding fraction obtained from a culture supernatant which was from a lot different from the lot used for mass spectrometry analysis. The fraction was separated by electrophoresis (Bio Rad Ready Gel (5 to 15%)) and transferred to a PVDF membrane (9 V, 16.5 hours (about 150 Vhr)). A hamster anti-mouse MFG-E8 antibody (MBL International Corporation) was used as a primary antibody for the reaction at 1 µg/ml for 2 hours (room temperature) and a HRP-conjugated goat anti-hamster IgG (Santa Cruz Biotechnology, Inc.) at a 1000-fold dilution was used as a secondary antibody for the reaction for 1 hour (room temperature). The detection was carried out with an exposing agent from PIERCE for an exposure period of 3 minutes.

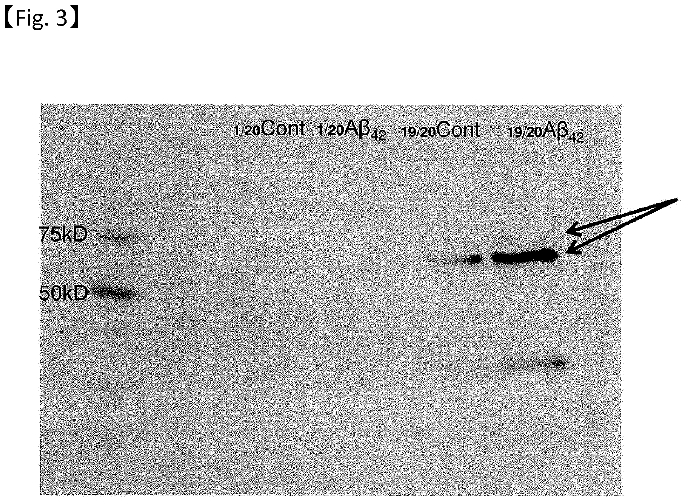

The results are shown in Fig. 3. The obtained signal was considered to correspond to the MFG-E8 long form (isoform 1) based on its molecular weight. The significant difference was also observed at a protein level, where the band detected in the lane for the sample stimulated with Aβ42 (0.1 µM) was more intense than the band detected for the control (without stimulation).

### Example 3

### Measurement of MFG-E8 concentration in plasma of patients with Alzheimer's disease

The MFG-E8 concentration was measured by ELISA in the plasma of patients with Alzheimer's disease (AD) and healthy elderly subjects at the corresponding ages.

Plasma was prepared from 20 healthy elderly subjects and 20 AD patients and ELISA was carried out with an anti-MFG-E8 antibody and an ELISA Kit for Human Milk Fat Globule EGF Factor 8 (USCN Life Science Inc.) following the procedures according to the instructions attached to the kit. The samples were diluted at 10 folds. After the concentration was measured, the statistical analysis was carried out with JMP.

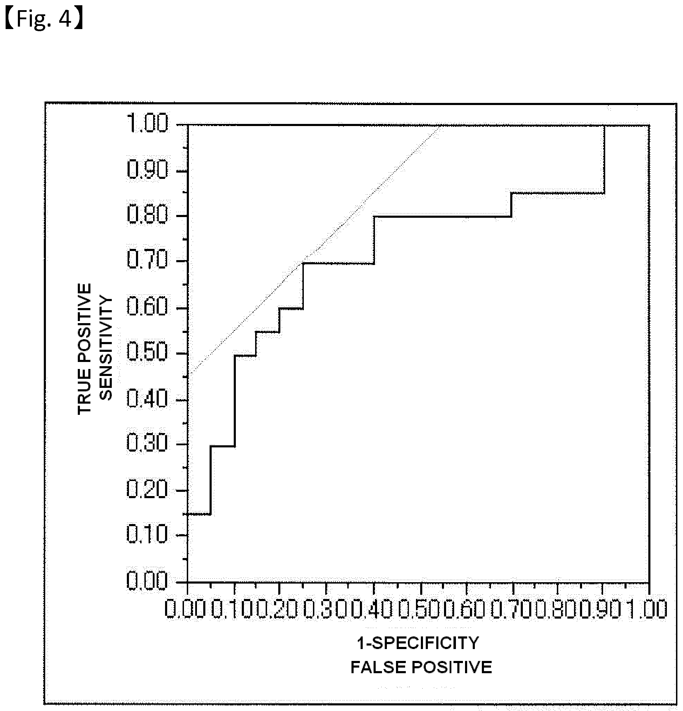

Fig. 4 shows the ROC curve of the MFG-E8 concentration in the plasma of healthy elderly subjects vs. AD patients (positive: AD). The area under the ROC curve was 0.72000, indicating a significant difference.

These results demonstrate that MFG-E8 was present in the culture medium of the mouse brain nerve cells stimulated with Aβ42 and was detected at a higher level in the plasma of AD patients than in that of healthy subjects.

### INDUSTRIAL APPLICABILITY

The present invention is useful for diagnosis of Alzheimer's disease.

## Claims

1. A method for diagnosing Alzheimer's disease, comprising measuring the level of a MFG-E8 protein in a blood, serum or plasma sample to be obtained from a human or animal subject and comparing the resulting measured value to a measured value to be obtained from a healthy control, wherein the measured value of the subject higher than that of the control indicates that the subject has a high possibility of Alzheimer's disease.

2. The method according to claim 1, wherein the diagnosis of Alzheimer's disease is made at a stage where an accumulation of an amyloid β protein has not been advanced.

3. The method according to claim 1, wherein the level of the MFG-E8 protein is measured with an anti-MFG-E8 antibody.

4. Use of a diagnostic reagent comprising an anti-MFG-E8 antibody for a method according to claim 1.

5. Use of a test kit comprising an anti-MFG-E8 antibody and a reagent for detecting a MFG-E8 protein bound to the anti-MFG-E8 antibody for a method according to claim 1.

6. A method for selecting a candidate substance for a therapeutic agent for Alzheimer's disease, comprising the steps of:
administering a test substance to a model cell or a model animal of Alzheimer's disease;
measuring the amount of a MFG-E8 protein in a culture medium of the model cell or a sample obtained from the model animal; and
selecting the test substance as a candidate substance for a therapeutic agent for Alzheimer's disease when the amount of the MFG-E8 protein is decreased with administration of the test substance compared to the amount thereof without administration.

## Patentansprüche

1. Verfahren zur Diagnose der Alzheimer-Krankheit, umfassend das Messen des Niveaus eines MFG-E8-Proteins in einer Blut-, Serum- oder Plasmaprobe, die von einem menschlichen oder einem tierischen Individuum zu nehmen ist, und Vergleichen des erhaltenen Messwertes mit einem Messwert, welcher von einer gesunden Kontrolle zu erhalten ist, wobei ein höherer Messwert im Individuum verglichen mit der Kontrolle anzeigt, dass das Individuum eine hohe Wahrscheinlichkeit für die Alzheimer-Krankheit aufweist.

2. Verfahren nach Anspruch 1, **dadurch gekennzeichnet, dass** die Diagnose der Alzheimer-Krankheit in einem Stadium erfolgt, in dem keine Akkumulation eines Amyloid-β-Proteins erfolgt.

3. Verfahren nach Anspruch 1, **dadurch gekennzeichnet, dass** der Pegel des MFG-E8-Proteins mit einem Anti-MFG-E8-Antikörper gemessen wird.

4. Verwendung eines diagnostischen Reagens, umfassend einen Anti-MFG-E8-Antikörper für ein Verfahren nach Anspruch 1.

5. Verwendung eines Testkits, umfassend einen Anti-MFG-E8-Antikörper und ein Reagens zum Nachweis eines an den Anti-MFG-E8-Antikörper gebundenen MFG-E8-Proteins für ein Verfahren nach Anspruch 1.

6. Verfahren zur Selektion einer Kandidatenstoffsubstanz für ein therapeutisches Mittel für die Alzheimer-Krankheit, umfassend die Schritte:
Verabreichen einer Testsubstanz an eine Modellzelle oder ein Modelltier der Alzheimer-Krankheit;
Messen der Menge eines MFG-E8-Proteins in einem Kulturmedium der Modellzelle oder einer aus dem Modelltier erhaltenen Probe; und
Auswählen der Testsubstanz als Kandidatenstoff für ein therapeutisches Mittel für die Alzheimer-Krankheit, wenn die Menge des MFG-E8-Proteins mit der Verabreichung der Testsubstanz im Vergleich zu ihrer Menge ohne Verabreichung verringert wird.

## Revendications

1. Procédé pour diagnostiquer la maladie d'Alzheimer, comprenant la mesure du niveau d'une protéine MFG-E8 dans un échantillon de sang, de sérum ou de plasma à prélever chez un sujet humain ou animal, et la comparaison de la valeur mesurée résultante à une valeur mesurée à obtenir à partir d'un témoin sain, dans lequel la valeur mesurée du sujet plus élevée que celle du témoin indique que le sujet présente une forte possibilité de maladie d'Alzheimer.

2. Procédé selon la revendication 1, dans lequel le diagnostic de la maladie d'Alzheimer est effectué à un stade où une accumulation d'une protéine amyloïde β n'a pas été avancée.

3. Procédé selon la revendication 1, dans lequel le niveau de la protéine MFG-E8 est mesuré avec un anticorps anti-MFG-E8.

4. Utilisation d'un réactif de diagnostic comprenant un anticorps anti-MFG-E8 pour un procédé selon la revendication 1.

5. Utilisation d'un kit de test comprenant un anticorps anti-MFG-E8 et un réactif pour détecter une protéine MFG-E8 liée à l'anticorps antiMFG-E8 pour un procédé selon la revendication 1.

6. Procédé de sélection d'une substance candidate pour un agent thérapeutique destiné à la maladie d'Alzheimer, comprenant les étapes de :
administration d'une substance test à une cellule modèle ou un animal modèle de la maladie d'Alzheimer ;
mesure de la quantité d'une protéine MFG-E8 dans un milieu de culture de la cellule modèle ou un échantillon prélevé chez l'animal modèle ; et
sélection de la substance test en tant que substance candidate pour un agent thérapeutique destiné à la maladie d'Alzheimer lorsque la quantité de la protéine MFG-E8 est réduite par l'administration de la substance test par rapport à la quantité de celle-ci sans l'administration.
